# EUROPEAN PATENT SPECIFICATION

(11) **EP 0 724 639 B1**
(45) Date of publication and mention of the grant of the patent: **31.01.2001**
(21) Application number: 92908635.3
(22) Date of filing: 20.02.1992
(51) Int. Cl.: C12N 15/00, C12N 15/67, C12N 15/85

(54) **METHODS FOR SELECTION OF RECOMBINANT HOST CELLS EXPRESSING HIGH LEVELS OF A DESIRED PROTEIN**
METHODEN ZUR SELEKTION VON REKOMBINANTEN WIRTSZELLEN WELCHE HOHE MENGEN VON EINEM GEWÜNSCHTEN PROTEIN EXPRIMIEREN
PROCEDES POUR LA SELECTION DE CELLULES HOTES DE RECOMBINAISON EXPRIMANT DES NIVEAUX ELEVES D'UNE PROTEINE DESIREE

(30) Priority: 29.03.1991 US 677045
(43) Date of publication of application: 07.08.1996
(73) Proprietor: GENENTECH, INC., South San Francisco California 94080 (US)
(72) Inventor: COHEN, Justus, B., Allison Park, PA 15101 (US)
(74) Representative: Barz, Peter, Dr.
(86) International application number: US9201358
(87) International publication number: WO9217566

(56) References cited:
- WO-A-90/12025
- MOL. CELL. BIOL. vol. 8, no. 10, October 1988, AM. SOC.MICROBIOL. WASHINGTON,DC,US; pages 3495 - 4405 A.R. BUCHMAN AND P. BERG 'Comparison of intron-dependent and intron-independent gene expression' the whole document
- BIO/TECHNOLOGY vol. 9, no. 1, January 1991, NATURE AMERICA, INC., NEW YORK, US pages 64 - 68 M.J. PAGE AND M.A. SYDENHAM 'High level expression of the humanized monoclonal antibody campath-1H in chinese hamster ovary cells' cited in the application the whole document

## Description

### Background of the Invention

The discovery of methods for introducing DNA into living host cells in a functional form has provided the key to understanding many fundamental biological processes, and has made possible the production of important proteins and other molecules in commercially useful quantities.

Despite the general success of such gene transfer methods, several common problems exist that may limit the efficiency with which a gene encoding a desired protein can be introduced into and expressed in a host cell. One problem is knowing when the gene has been successfully transferred into recipient cells. A second problem is distinguishing between those cells that contain the gene and those that have survived the transfer procedures but do not contain the gene. A third problem is identifying and isolating those cells that contain the gene and that are expressing high levels of the protein encoded by the gene.

In general, the known methods for introducing genes into eukaryotic cells tend to be highly inefficient. Of the cells in a given culture, only a small proportion take up and express exogenously added DNA, and an even smaller proportion stably maintain that DNA.

Identification of those cells that have incorporated a product gene encoding a desired protein typically is achieved by introducing into the same cells another gene, commonly referred to as a selectable gene, that encodes a selectable protein. A selectable protein is one that is necessary for the growth or survival of a host cell under the particular culture conditions chosen, such as an enzyme that confers resistance to an antibiotic or other drug, or an enzyme that compensates for a metabolic or catabolic defect in the host cell. For example, selectable genes commonly used with eukaryotic cells include the genes for aminoglycoside phosphotransferase IAPH or neo), hygromycin phosphotransferase (hyg), dihydrofolate reductase (DHFR), and thymidine kinase (tk).

The method of identifying a host cell that has incorporated one gene on the basis of expression by the host cell of second incorporated gene encoding a selectable protein is referred to as cotransfection (also cotransformation). In that method, a product gene encoding a desired protein and a selectable gene are introduced into the host cell simultaneously. The product gene and the selectable gene may be present on a single DNA molecule or on separate DNA molecules prior to being introduced into the host cells. Wigler, et al., Cell 16:777 (1979). Cells that have incorporated and express the selectable gene then are identified or isolated by culturing under conditions that allow for the growth or survival of only those cells that synthesize the selectable protein encoded by the selectable gene. Typically, the product gene is present and expressed in many of the cells thus identified or isolated.

The level of expression of a gene introduced into a eukaryotic host cell depends on multiple factors, including gene copy number, efficiency of transcription, messenger RNA (mRNA) processing, stability, and translation efficiency. Accordingly, high-level expression of a desired protein typically will involve optimizing one or more of those factors.

For example, the level of protein production may be increased by covalently joining the coding sequence of the gene to a "strong" promoter or enhancer that will give high levels of transcription. Promoters and enhancers are nucleotide sequences that interact specifically with proteins in a host cell that are involved in transcription. Kriegler, Meth. Enzymol. 185:512 (1990); Maniatis, et al., Science 236:1237 (1987). Promoters are nucleotide sequences that are located upstream of the coding sequence of a gene and that facilitate transcription of the gene by RNA polymerase. Among the eukaryotic promoters that have been identified as strong promoters for high-level expression are the SV40 early promoter, adenovirus major late promoter, mouse metallothionein-I promoter, Rous sarcoma virus long terminal repeat, and human cytomegalovirus (CMV) major immediate-early promoter.

Enhancers stimulate transcription from a linked promoter. Unlike promoters, enhancers are active when placed downstream from the transcription initiation site or at considerable distances from the promoter, although in practice enhancers may overlap physically and functionally with promoters. For example, all of the strong promoters listed above also contain strong enhancers. Bendig, Genetic Engineering 7:91 (Academic Press, 1988).

The level of protein production also may be increased by increasing the gene copy number in the host cell. One method for obtaining high gene copy number is to directly introduce into the host cell multiple copies of the gene, for example, by using a large molar excess of the product gene relative to the selectable gene during contransfection. Kaufman, Meth. Enzymol. 185:537 (1990). With this method, however, only a small proportion of the cotransfected cells will contain the product gene at high copy number, and because no generally applicable, convenient method exists for distinguishing such cells from the majority of cells that contain fewer copies of the product gene, laborious and time-consuming screening methods typically are required to identify the desired high copy number transfectants.

Another method for obtaining high gene copy number involves cloning the gene in a vector that is capable of replicating autonomously in the host cell. Examples of such vectors include mammalian expression vectors derived from Epstein-Barr virus or bovine papilloma virus, and yeast 2-micron plasmid vectors. Stephens & Hentschel, Biochem. J. 248:1 (1987); Yates, et al., Nature 313:812 (1985); Beggs, Genetic Engineering 2:175 (Academic Press, 1981). The usefulness of this method is limited, however, because uncontrolled replication of such vectors is incompatible with cell viability, and controlled replication limits the vector copy number. Furthermore, autonomous replication has been shown to interfere with gene expression, probably at the level of transcription. Lebkowski, et al., Nature 317:169 (1985).

Yet another method for obtaining high gene copy number involves gene amplification in the host cell. Gene amplification occurs naturally in eukaryotic cells at a relatively low frequency. Schimke, J. Biol. Chem. 263:5989 (1988). However, gene amplification also may be induced, or at least selected for, by exposing host cells to appropriate selective pressure. For example, in many cases it is possible to introduce a product gene together with a selectable and amplifiable marker gene into a host cell and subsequently select for amplification of the marker gene by exposing the cotransfected cells to sequentially increasing concentrations of a selective agent. Typically the product gene will be coamplified with the marker gene under such conditions.

The most widely used selectable and amplifiable marker gene for that purpose is a DHFR gene. The selective agent used in conjunction with a DHFR gene is methotrexate (Mtx). A host cell is cotransfected with a product gene encoding a desired protein and a DHFR gene, and transfectants are identified by first culturing the cells in culture medium that contains Mtx. A suitable host cell when a wild-type DHFR gene is used is the Chinese Hamster ovary (CHO) cell line deficient in DHFR activity, prepared and propagated as described by Urlaub & Chasin, Proc. Nat. Acad. Sci. 77:4216 (1980). The transfectant cells then are exposed to successively increasing amounts of Mtx. This leads to the synthesis of multiple copies of the DHFR gene, and concomitantly, multiple copies of the product gene. Schimke, J. Biol. Chem. 263:5989 (1988); Axel, et al., U.S. Patent No. 4,399,216; Axel, et al., U.S. Patent No. 4.634,665.

To extend the DHFR amplification method to other cell types, a mutant DHFR gene that encodes a protein with reduced sensitivity to methotrexate may be used in conjunction with host cells that contain normal numbers of an endogenous wild-type DHFR gene. Simonsen & Levinson, Proc. Nat. Acad. Sci. 80:2495 (1983). Alternatively, host cells may be cotransfected with the product gene, a DHFR gene, and a dominant selectable gene, such as a neo' gene. Kim & Wold, Cell 42:129 (1985). Transfectants are identified by first culturing the cells in culture medium containing neomycin (or the related drug G418), and the transfectants so identified then are selected for amplification of the DHFR gene and the product gene by exposure to successively increasing amounts Mtx.

As will be appreciated from this discussion, the selection of recombinant host cells that express high levels of a desired protein generally is a multi-step process. In the first step, initial transfectants are selected that have incorporated the product gene and the selection gene. In subsequent steps, the initial transfectants are subject to further selection for high-level expression of the selectable gene and then random screening for high-level expression of the product gene. To identify cells expressing high levels of the desired protein, typically it is necessary to screen large numbers of transfectants. The majority of transfectants produce less than maximal levels of the desired protein.

Several methods have been described for directly selecting such recombinant host cells in a single step. For example, one strategy involves cotransfecting host cells with a product gene and a DHFR gene, and selecting those cells that express high levels of DHFR by directly culturing in medium containing a high concentration of Mtx. Many of the cells selected in that manner also express the cotransfected product gene at high levels. Page & Sydenham, Bio/Technology 9:64 (1991). Another method involves the use of polycistronic mRNA expression vectors containing a product gene at the 5' end of the transcribed region and a selectable gene at the 3' end. Because translation of the selectable gene at the 3' end of the polycistronic mRNA is inefficient, such vectors exhibit preferential translation of the desired gene and require high levels of polycistronic mRNA to survive selection. Kaufman, Meth. Enzymol. 185:487 (1990); Kaufman, Meth. Enzymol. 185:537 (1990); Kaufman, et al., EMBO J. 6:187 (1987). Accordingly, cells expressing high levels of the desired protein product may be obtained in a single step by culturing the initial transfectants in medium containing a selective agent appropriate for use with the particular selectable gene.

Unfortunately, those known methods for single-step selection suffer from certain drawbacks which limit their usefulness. High expressing cells obtained by direct culturing in medium containing a high level of a selective agent may have poor growth and stability characteristics, thus limiting their usefulness for long-term production processes. Page & Snyderman, Bio/Technology 9:64 (1991). Single-step selection for high-level resistance to Mtx may produce cells with an altered, Mtx-resistant DHFR enzyme, or cells that have altered Mtx transport properties, rather than cells containing amplified genes. Haber, et al., J. Biol. Chem. 256:9501 (1981); Assaraf & Schimke, Proc. Nat. Acad. Sci. 84:7154 (1987). With polycistronic vectors, there is a strong selection for deletion or rearrangement of the 5' product gene when selecting for expression of the 3' selectable gene. Cells harboring vectors having such mutations in the 5' product gene tend to outgrow other cells in the population, and thus interfere with the selection of cells actually expressing high levels of the desired protein. Kaufman, Meth. Enz. 185:537 (1990).

It is one object of the present invention, therefore, to provide a method for obtaining high-level expression of a desired protein product in recombinant host cells that does not involve exposure of the cells to high concentrations of Mtx or another selective agent, or the use of autonomously replicating vectors, or polycistronic mRNA expression vectors. It is another object of the present invention to provide a method for selecting recombinant host cells that express high levels of a desired protein product, which method is rapid and convenient to perform, and does not require screening of large numbers of cells. It is another object of the present invention to provide a new method for single-step selection of recombinant host cells that express high levels of a desired protein product.

The present invention thus provides improved methods for the selection of recombinant host cells expressing high levels of a desired protein, which methods are useful with a wide variety of eukaryotic host cells and avoid the problems inherent in existing cell selection technology.

### Summary of the Invention

The present invention is directed to improved methods for selection of recombinant host cells expressing high levels of a desired protein. In the methods of the present invention, a starting selectable gene is modified by inserting into its transcribed region an intron of such length that the intron is correctly spliced from the corresponding mRNA precursor at low efficiency. As a consequence thereof, the amount of selectable protein produced from the intron-modified selectable gene is substantially less than that produced from the starting selectable gene.

Host cells then are cotransfected with the intron-modified selectable gene and a product gene encoding a desired protein. After such cotransfection, it is observed that most of the resulting transfectants fail to exhibit the selectable phenotype that is characteristic of the selectable protein, as a consequence of the relatively low level of selectable protein that is produced from the intron-modified selectable gene in the transfectants. Surprisingly, however, a small proportion of the transfectants do exhibit the selectable phenotype, and among those transfectants, the majority are found to express high levels of the desired protein encoded by the product gene.

### Brief Description of the Drawings

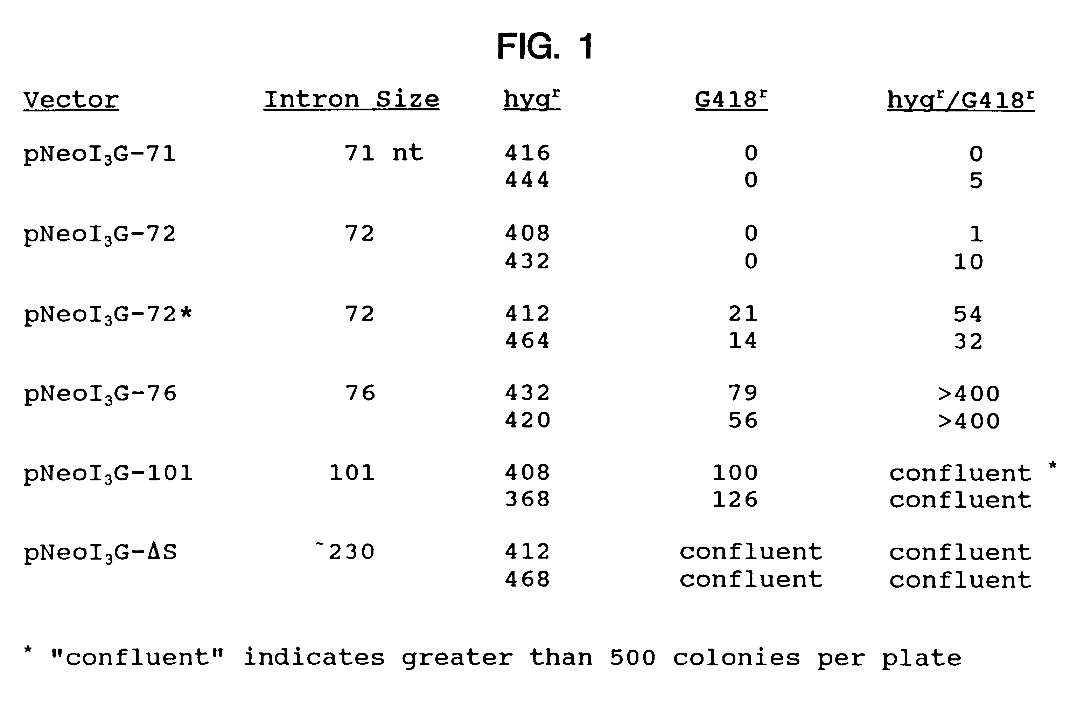

Figure 1 shows the number of colonies of hygromycin-resistant (hyg'), neomycin-resistant (neo'), and hygromycin/neomycin-resistant (hyg'/neo') cells obtained after cotransfecting Ras 2.2 cells as described in Example 2. The length of the synthetic intron present within each different neomycin resistance gene is indicated. The construction of vectors containing the neomycin resistance genes is described in Example 1.

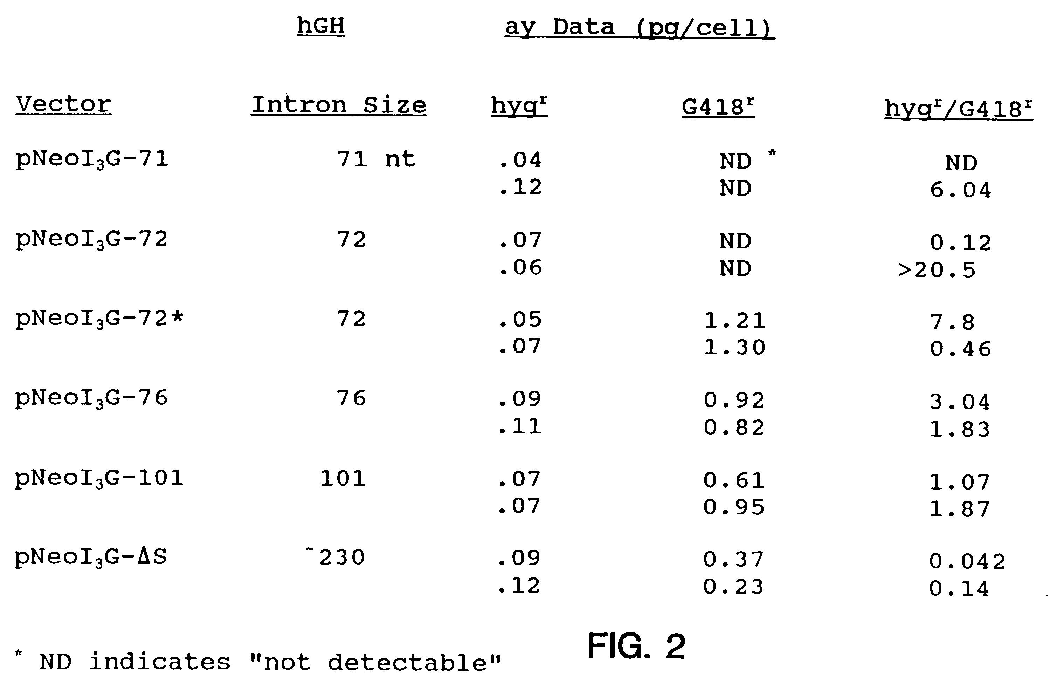

Figure 2 shows the levels of human growth hormone (hGH) produced by various drug-resistant cell cultures. Ras 2.2 cells were cotransfected with the vectors as described in Example 2. Levels of hGH (pg/cell) in the medium from drug-resistant cell cultures were determined by an ELISA radioimmunoassay.

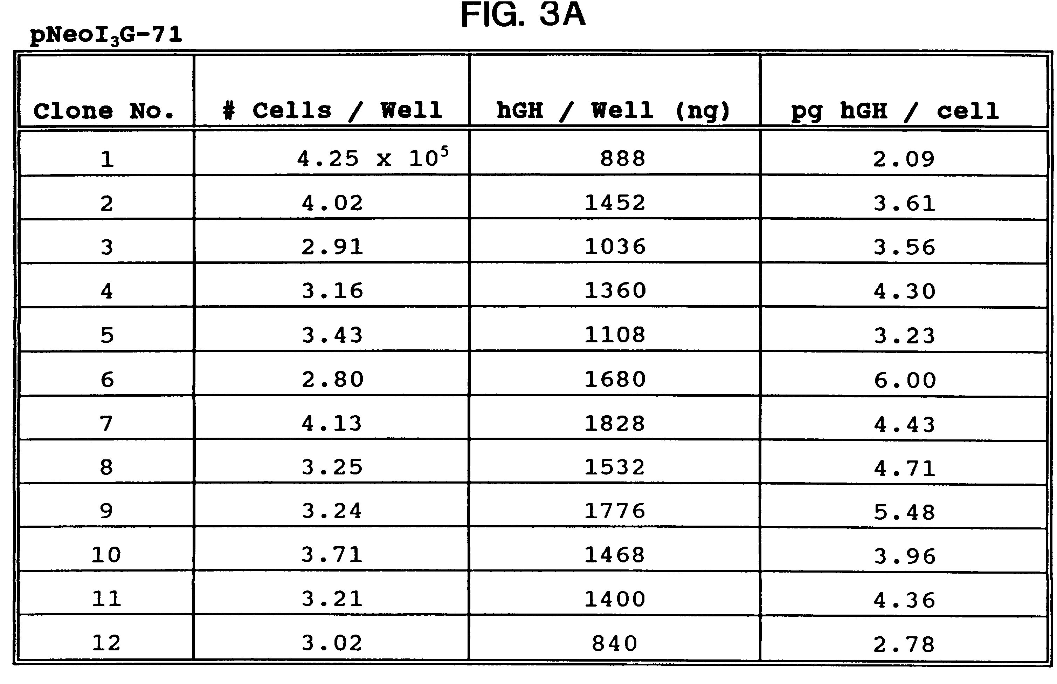

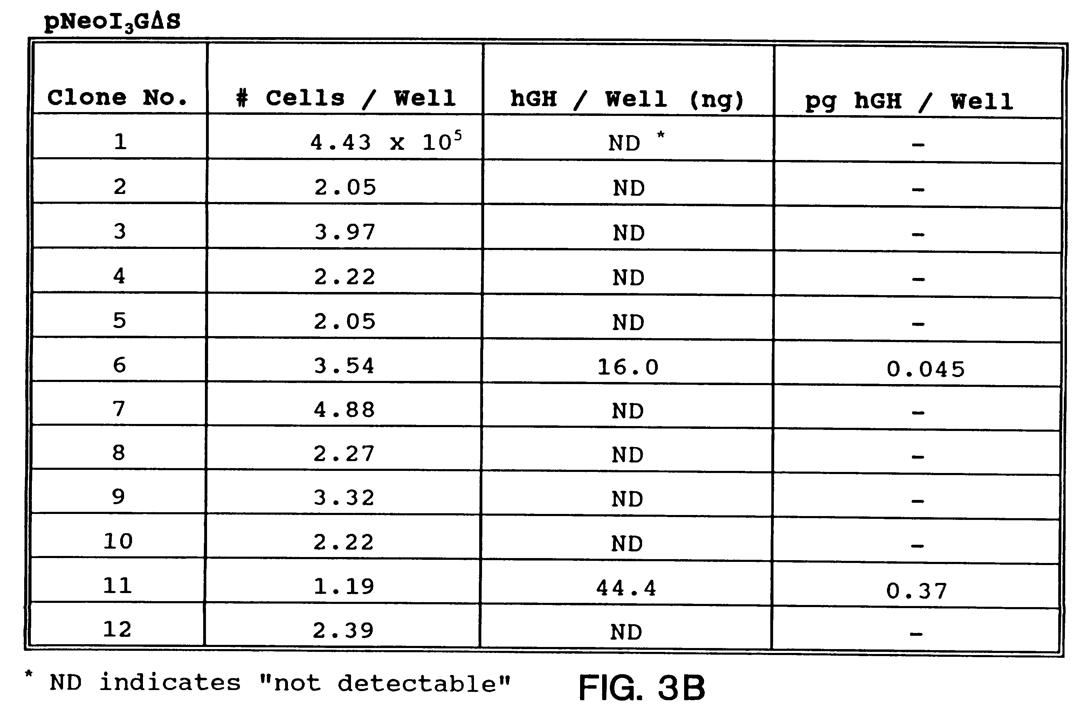

Figure 3 shows the levels of hGH produced by individual cell clones obtained from cultures of Ras 2.2 cells transfected with pRK-hyg, pRSV-hGH, and either pNeol₃G-71 or pNeol₃GΔS, as described in Example 2.

### Detailed Description of the Invention

In general, the methods of the present invention involve cotransfecting a eukaryotic host cell with a product gene encoding a desired protein and with a selectable gene that includes an intron, which intron reduces the level of selectable protein produced from the selectable gene in the host cell, as compared to the selectable gene without that intron.

Introns are noncoding nucleotide sequences, normally present within many eukaryotic genes, which are removed from newly transcribed mRNA precursors in a multiple-step process collectively referred to as splicing.

A single mechanism is thought to be responsible for the splicing of mRNA precursors in mammalian, plant, and yeast cells. In general, the process of splicing requires that the 5' and 3' ends of the intron be correctly cleaved and the resulting ends of the mRNA be accurately joined, such that a mature mRNA having the proper reading frame for protein synthesis is produced.

The positions at which cleavage of the intron occurs are referred to as splice sites. By comparing the nucleotide sequences that surround the splice sites in a large number of different genes, it has been possible to define consensus sequence for the splice site at each end of an intron. The consensus sequence specifies the most common nucleotide found at each position relative to the splice site. For example, in the mRNAs of higher eukaryotes, the 5' splice site occurs within the consensus sequence AG:GUAAGU (wherein the colon denotes the site of cleavage and ligation), and the 3' splice site occurs within the consensus sequence (U/C)ₙNCAG:G. In the mRNAs of yeast, the 5' splice site is bounded by the consensus sequence :GUAUGU, and the 3' splice site is bounded by the consensus sequence (C/U)AG:. Ohshima & Gotoh, J. Mol. Biol. 195:247 (1987); Padgett, et al., Ann. Rev. Biochem. 55:1119 (1986); Mount, Nuc. Acids Res. 10:459 (1982).

Analysis of a variety of naturally occurring and synthetically constructed mutant genes has shown that nucleotide changes at many of the positions within the consensus sequences at the 5' and 3' splice sites have the effect of reducing or abolishing the synthesis of mature mRNA. Sharp, Science 235:766 (1987); Padgett, et al., Ann. Rev. Biochem. 55:1119 (1986); Green, Ann. Rev. Genet. 20:671 (1986). Mutational studies also have shown that RNA secondary structure involving splicing sites can affect the efficiency of splicing. Solnick, Cell 43:667 (1985); Konarska, et al., Cell 42:165 (1985).

The length of an intron also may affect the efficiency of splicing. By making deletion mutations of different sizes within the large intron of the rabbit beta-globin gene, Wieringa, et al. determined that the minimum intron length necessary for correct splicing is about 69 nucleotides (nts). Cell 37:915 (1984). Similar studies of the intron of the adenovirus E1A region have shown that an intron length of about 78 nts allows correct splicing to occur, but at reduced efficiency. Increasing the length of the intron to 91 nts restored normal splicing efficiency, whereas truncating the intron to 63 nts abolished correct splicing. Ulfendahl, et al., Nuc. Acids Res. 13:6299 (1985).

In one of its embodiments, the present invention provides a method for identifying or isolating a recombinant host cell that expresses high levels of a desired protein, which method comprises the steps of:
(1) cotransfecting a eukaryotic host cell with
   (a) a selectable gene modified to contain an intron not present in the naturally occurring selectable gene, wherein the intron is capable of being spliced in said host cell to provide messenger RNA encoding a selectable protein and wherein the presence of the intron in the selectable gene reduces the level of selectable protein produced from the selectable gene in the host cell, and
   (b) a gene encoding a desired protein; and
(2) culturing the resulting transfectants in medium containing a selective agent appropriate for the selectable gene.

In another of its embodiments, the present invention provides a method for identifying or isolating a recombinant host cell that expresses high levels of a desired protein, which method comprises the steps of:
(1) cotransfecting a eukaryotic host cell with
   (a) a first selectable gene,
   (b) a second selectable gene, modified to contain an intron not present in the naturally occurring selectable gene, wherein the intron is capable of being spliced in said host cell to provide messenger RNA encoding a selectable protein and wherein the presence of the intron in the selectable gene reduces the level of selectable protein produced from the selectable gene in the host cell, and
   (c) a gene encoding a desired protein;
(2) culturing the resulting transfectants in medium containing a selective agent appropriate for the first selectable gene; and
(3) culturing the transfectants that survive the selection of step (2) in medium containing a selective agent appropriate for the second selectable gene.

The term "selectable gene" as used herein refers to a DNA that encodes a protein necessary for the growth or survival of a host cell under the particular cell culture conditions chosen. Accordingly, a host cell that is transformed with a selectable gene will be capable of growth or survival under certain cell culture conditions wherein a non-transfected host cell is not capable of growth or survival. Typically, a selectable gene will confer resistance to a drug or compensate for a metabolic or catabolic defect in the host cell. For example, commonly used selection genes include the genes for aminoglycoside phosphotransferase (APH or neo), hygromycin phosphotransferase (hyg), dihydrofolate reductase (DHFR), and thymidine kinase (tk).

The term "selectable protein" as used herein refers to a protein encoded by a selectable gene. The term "selectable phenotype" refers to the phenotype conferred on a host cell by a selectable gene or a selectable protein. The term "selection agent" refers to a substance that interferes with the growth or survival of a host cell that is deficient in a particular selectable marker. For example, in the case of a selectable gene that encodes aminoglycoside phosphotransferase, suitable selection agents include neomycin and G418. In the case of a selectable gene that encodes hygromycin phosphotransferase, a suitable selection agent is hygromycin.

The term "product gene" as used herein refers to a DNA that encodes a desired protein product. Any product gene which is capable of expression in a host cell may be used, although the methods of the invention are particularly suited for obtaining high level expression of a product gene that is not also a selectable gene. Accordingly, the protein encoded by a product gene typically will be one that is not necessary for the growth or survival of a host cell under the particular cell culture conditions chosen. For example, product genes may encode antibodies, viral antigens, interferons, or growth hormones.

Selectable genes and product genes may be obtained from genomic DNA, cDNA transcribed from cellular RNA, or by in vitro synthesis. For example, the isolation of such genes from genomic DNA or cDNA libraries is conveniently accomplished by the use of a DNA hybridization probe that is labeled with a detectable moiety, such as a radioisotope, and that is capable of hybridizing preferentially to a desired selectable gene or product gene through complementary base-pairing. Keller & Manak, DNA Probes, pp.149-213 (1989). Alternatively, a desired selectable gene or a product gene may be obtained by using the polymerase chain reaction (PCR) method to amplify such a gene that is present within the nucleic acid of a suitable viral, cell, or tissue source. Preferably, the product gene and the intron-modified selectable gene used in the methods of the present invention are on separate DNA molecules, so that a large molar excess of the product gene relative to the intron-modified selectable gene can be used for cotransfection.

The term "intron" as used herein refers to a nucleotide sequence present within the transcribed region of a gene or within a messenger RNA precursor, which nucleotide sequence is capable of being excised, or "spliced", from the messenger RNA precursor by a host cell prior to translation. Introns suitable for use in the present invention may be prepared by any of several methods that are well known in the art, such as purification from a naturally occurring nucleic acid or de novo synthesis. The introns present in many naturally occurring eukaryotic genes have been identified and characterized. Mount, Nuc. Acids Res. 10:459 (1982). Artificial introns comprising functional splice sites also have been described. Winey, et al., Mol. Cell. Biol. 9:329 (1989); Gatermann, et al., Mol. Cell Bio. 9:1526 (1989). Introns may be obtained from naturally occurring nucleic acids, for example, by digestion of a naturally occurring nucleic acid with a suitable restriction endonuclease, or by PCR cloning using primers complementary to sequences at the 5' and 3' ends of the intron. Alternatively, introns of defined sequence and length may be prepared synthetically using various methods in organic chemistry. Narang, et al., Meth. Enzymol. 68:90 (1979); Caruthers, et al., Meth. Enzymol. 154:287 (1985); Froehler, et al., Nuc. Acids Res. 14:5399 (1986).

The terms "PCR" and "polymerase chain reaction" as used herein refer to the in vitro amplification method described in U.S. Patent No. 4,683,195 (issued July 28, 1987). In general, the PCR method involves repeated cycles of primer extension synthesis, using two DNA primers capable of hybridizing preferentially to a template nucleic acid comprising the nucleotide sequence to be amplified. The PCR method can be used to clone specific DNA sequences from total genomic DNA, cDNA transcribed from cellular RNA, viral or plasmid DNAs. Wang & Mark, in PCR Protocols, pp.70-75 (Academic Press, 1990); Scharf, in PCR Protocols, pp.84-98; Kawasaki & Wang, in PCR Technology, pp.89-97 (Stockton Press, 1989).

A selectable gene may be modified to contain an intron not normally present within the selectable gene using any of the various known methods for modifying a nucleic acid in vitro. Typically, an intron will be introduced into a selectable gene by first cleaving the selectable gene with a restriction endonuclease within a region of the gene that normally is transcribed into RNA, and then covalently joining the resulting restriction fragments to the intron in the correct orientation for host cell expression, for example by ligation with a DNA ligase enzyme.

To be useful in the invention, the intron that is introduced into the selectable gene must have a length such that the level of expression of the intron-modified selectable gene in the host cell is substantially reduced as compared to the unmodified selectable gene. Preferably the intron will have the minimum length that is possible without abolishing splicing of the intron and thus the synthesis of mature mRNA encoding the selectable protein. Typically, the intron that is introduced into the selectable gene will have a length of between 50 and 80 nts, although it may be somewhat smaller or larger.

Since it is difficult to predict in advance precisely what size intron will be optimal within a particular selectable gene in a particular host cell for obtaining high-level expression of a product gene, it will be appreciated that some screening of different size introns will be necessary. Once the proper size intron is determined, however, it can be used routinely in the methods of the present invention to obtain high-level expression of any desired protein. The length of the intron should be such that relatively few initial transfectants are obtained that exhibit the selectable phenotype characteristic of the selectable protein encoded by the intron-modified selectable gene, as compared to the number of initial transfectants obtained when the length of the intron is increased by one or a few (preferably less than 10) nucleotides.

The preparation of introns of differing lengths is a routine matter, involving methods well known in the art, such as de novo synthesis or in vitro deletion mutagenesis of an existing intron. Likewise, the determination of the levels of expression of a product gene in various host cells containing selectable genes modified by introns of differing lengths is a routine matter, involving analytical methods well known in the art, such as analytical gel electrophoresis, ELISA assay, or radioimmunoassay.

The term "expression" as used herein refers to transcription or translation occurring within a host cell. The level of expression of a product gene in a host cell may be determined on the basis of either the amount of corresponding mRNA that is present in the cell or the amount of the protein encoded by the product gene that is produced by the cell. For example, mRNA transcribed from a product gene is can be quantitated by northern hybridization. Sambrook, et al., Molecular Cloning: A Laboratory Manual, pp.7.3-7.57 (Cold Spring Harbor Laboratory Press, 1989). Protein encoded by a product gene can be quantitated either by assaying for the biological activity of the protein or by employing assays that are independent of such activity, such as western blotting or radioimmunoassay using antibodies that are capable of reacting with the protein. Sambrook, et al., Molecular Cloning: A Laboratory Manual, pp.18.1-18.88 (Cold Spring Harbor Laboratory Press, 1989).

For transcription of a gene, whether a selectable gene, an intron-modified selectable gene, or a product gene encoding a desired protein, it is necessary that the gene be operably linked to a promoter functional in the particular host cell used for expression. A gene is operably linked to a promoter when it is capable of being transcribed into RNA under the control of the promoter.

Promoters suitable for use in mammalian host cells include the SV40 early promoter, adenovirus major late promoter, mouse metallothionein-I promoter, Rous sarcoma virus LTR promoter, human cytomegalovirus major immediate-early promoter, mouse mammary tumor virus LTR promoter, and herpes simplex virus thymidine kinase promoter. Sambrook, et al., Molecular Cloning: A Laboratory Manual, pp.16.5-16.26 (Cold Spring Harbor Laboratory Press, 1989); Bendig, Genetic Engineering 7:91 (Academic Press, 1988). Promoters suitable for use in plant host cells include the promoters for octopine synthase (ocs) and nopaline synthase (nos) genes, and the 35S cauliflower mosaic virus promoter. Lichtenstein & Fuller, Genetic Engineering 6:104 (Academic Press, 1987). Promoters suitable for use in yeast host cells include the promoters for phosphoglycerate kinase (pgk), enolase (eno), alcohol dehydrogenase (adh), and other glycolytic genes. Kinsman & Kingsman, Genetic Engineering, pp.76-83 (Blackwell Scientific Publications, 1988).

The terms "transfection" and "cotransfection" as used herein refer generally to the process of introducing nucleic acid into a host cell. Various methods are known for introducing a gene into a eukaryotic host cell. The most commonly used of those methods involve direct DNA transfer into cells. For example, DNA may be introduced into mammalian cells by exposing the cells to DNA in the presence of calcium phosphate precipitate or DEAE-dextran. Keown, et al., Meth. Enzymol. 185:527 (1990). In contrast to mammalian cells, yeast and plant cells are surrounded by a thick wall which is relatively resistant to severe chemical and physical treatments and inhibits the uptake of many small molecules. Accordingly, the methods for introducing DNA into such cells typically have as their first step the conversion of the yeast or plant cells to spheroplasts or protoplasts, respectively, by removal of some of the cell wall. DNA is then introduced into the cell by exposing the spheroplasts or protoplasts to DNA in the presence of polyethylene glycol (PEG). Hinnen, et al., Proc. Nat Acad. Sci. 75:1929 (1978). Peerbolte, et al, Plant. Molec. Biol. 5:235 (1984); Potrykus, et al., Mol. Gen. Genet. 199:169 (1985). Alternatively, DNA may be introduced into intact yeast cells that have been treated with lithium acetate. Stearns, et al., Meth. Enzymol. 185:280 (1990).

Other methods for introducing genes into eukaryotic cells are well known in the art, such as those that involve electroporation, microinjection, or infection with recombinant viruses. Kaufman, Meth. Enzymol. 185:487 (1990); Keown, et al., Meth. Enzymol. 185:527 (1990); Lichtenstein, et al., Genetic Engineering 6:104 (Academic Press, 1987).

After cotransfection with a selectable gene modified to contain an intron as described above and a product gene encoding a desired protein, host cells are cultured in nutrient medium containing a selective agent appropriate for the particular selectable gene. Suitable nutrient media and cell culture methods are well known in the art. Sherman, Meth. Enzymol. 194:3 (1991); Mather, Meth. Enzymol. 185:567 (1990); Berlin & Bode, in Basic Biotechnology, pp.133-177 (VCH Publishers, 1987). Provided that the intron size is properly chosen, only a few initial transfectants will be obtained as a result of such single-step selection, which initial transfectants are conveniently assayed for high-level expression of the product gene.

Alternatively, host cells may be cotransfected with two selectable genes, encoding different selectable proteins, and a product gene encoding a desired protein. After transfection, the cells are cultured in nutrient medium containing a selective agent appropriate for the first selectable gene, and individual initial transfectants are selected. Subsequently, those initial transfectants are cultured in nutrient medium containing a selective agent appropriate for the second selectable gene, which second selectable gene is modified by an intron, as described above. As shown in Figure 2, by this two-step selection method, it may be possible to obtain substantially higher level expression of the product gene than is possible with the single-step selection method described above.

In accordance with the teachings of the present invention, therefore, high-level expression of a product gene encoding a desired protein is obtained by employing for expression a host cell that 1) is cotransfected with a selectable gene modified by an intron that reduces the amount of selectable protein produced from the selectable gene in the host cell, and 2) is thereafter capable of growth or survival under selective conditions that do not permit the growth or survival of the host cell that is lacking the selectable gene.

Accordingly, the methods of the present invention will be useful in the production of many different proteins in the industrial, agricultural, and pharmaceutical fields, and especially useful in the production of proteins that are available in limited quantities from natural sources, including such proteins as growth hormones, interferons, neurotrophic factors, DNase, erythropoietin, inhibin, insulin, relaxin, and tissue plasminogen activator. A desired protein that is produced in the recombinant host cells of the invention preferably will be recovered from the cell culture medium as a secreted protein, although it may also be recovered from host cell lysates. In either case, the desired protein can be purified from other host cell proteins by methods well known in the art to obtain preparations of the desired protein that are substantially homogeneous.

The following examples are offered by way of illustration only and are not intended to limit the invention in any manner.

### EXAMPLE I

### Construction of Vectors for Transfection

### 1. pSVE.hygB

The bacterial hygromycin B phosphotransferase gene ("hygB"), originally obtained in plasmid pLG90, Gritz & Davies, Gene 25:179 (1983), and modified with respect to the sequence immediately preceding the initiation codon as described by Cullen et al, Gene 57:21 (1987), was isolated as two restriction endonuclease fragments: a Clal-Pstl fragment containing the 5' portion of the gene and a Pstl-BamHI fragment containing the 3' portion. These fragments were ligated simultaneously to a large Clal-BamHl fragment containing the SV40 early enhancer/promoter and human hepatitis B virus (HBV) polyadenylation (poly(A)) regions in plasmid pML-1, Lusky & Botchan, Nature 293:79 (1981). The resulting vector contains the hygB gene preceded by the SV40 early enhancer/promoter and followed by the HBV poly(A) region.

### 2. pRK.hyg

Vector pRK.hyg was constructed by ligation of the large Hindlll-BamHl fragment of vector pRK7, PCT Pub. No. WO 90/02798 (published March 22, 1990), to the Hindlll-BamHl fragment of pSVE.hygB that contains the hygB coding sequence. Accordingly, the hygB gene in pRK.hyg is preceded by the enhancer/promoter and intron regions of pRK7 and followed by the SV40 late poly(A) region of pRK7.

### 3. pRSV-hGH

This vector contains the complete coding sequence for human growth hormone (hGH) under the control of transcriptional regulatory sequences from the long terminal repeat (LTR) of Rous sarcoma virus. It was constructed by substituting the ras promoter of rasP.hGH, Cohen & Levinson, Nature 334:119 (1988), with the RSV promoter.

### 4. pML-UM20

Plasmid pML-UM20 contains a polylinker region between the EcoRI and Hindlll sites of plasmid pML-1. It was constructed by ligating the large EcoRI-Hindlll fragment of pML-1 to the small EcoRI-Hindlll fragment of an intermediate plasmid, pUM20, which contains the polylinker region of plasmid pUC18, Norrander, et al., Gene 26:101 (1983). The sequence of the polylinker region in pML-UM20, including the EcoRI and Hindlll sites, is: This sequence includes recognition sites for the restriction endonucleases Smal (CCC/GGG), BamHI (G/GATCC), Sall (G/TCGAC) and Xhol (C/TCGAG) used in the constructions described below (cleavage sites are indicated by "/").

### 5. pSVI6B.neo

This vector contains a modified bacterial neomycin phosphotransferase (neo) gene preceded by the enhancer/promoter and intron regions of vector pSV16B5, PCT Pub. W091/08291 published 13 June 1991, and followed by the HBV poly(A) signal. It was constructed in several steps, as follows:

### a. Construction of intermediate plasmid pUC.neo3'

Plasmid pUC119 was linearized by digestion with Sphl and Pstl restriction endonucleases and then ligated simultaneously to (i) the Pstl-Smal fragment of vector pSVE.NeoBal6, Seeburg, et al., Nature 312:71 (1984), that contains the 3' portion of the neo coding region, and (ii) the BamHI-Sphl fragment of vector p311E, Liu et al., that contains the HBV poly(A) region. Prior to ligation, the BamHI cohesive end of the p311E-derived fragment was filled-in using the Klenow fragment of E.coli DNA polymerase I in the presence of all four deoxyribonucleotides to allow for its joining to the Smal-generated blunt end of the pSVE.NeoBal6-derived fragment. The resulting pUC.neo3' plasmid thus consists of the 3' end of the neo gene and the HBV poly(A) region inserted within the polylinker region of PUC119.

### b. Construction of pSVI6B.hyg

This vector was constructed through a three-part ligation joining (i) the SStl-Pstl fragment of vector pSVI6B.tPA that contains the enhancer/promoter and intron regions of that vector, and (ii) the Hindlll-Sall fragment of pSVI6B.tPA that contains the hygB coding sequence, to (iii) pUC119 linearized by digestion with Sstl and Sall. The 3' protruding end of the SVI6B.tPA fragment generated by Pstl cleavage was removed using T4 DNA polymerase, and the 5' protruding end of the SVI6B.tPA fragment generated by Hindlll digestion was filled-in to allow for blunt-end ligation of those two ends. The vector contains the SVI6B enhancer/promoter and intron regions, followed by the hygB coding sequence, followed by the HBV poly(A) region, inserted within the polylinker region of pUC119.

### c. Construction of SVI6B.neo

This vector was constructed by ligating (i) the large Sstl-PstI fragment of pUC.neo3', (ii) the Sstl-Clal fragment of pSVI6B.hyg that contains the SVI6B enhancer/promoter and intron regions, and (iii) the Clal-PstI fragment of pSVE.NeoBal6 that contains the 5' portion of the neo coding sequence.

### 6. pNeol₃G

Vector pNeol₃G differs from pSVI6B.neo by the presence of an intron within the neo coding sequence. The large Ball-Pstl fragment of pSVI6B.neo that contains all of pSVI6B.neo except for a short Ball-Pstl fragment internal to the neo gene was ligated to an intron-containing neo fragment that was generated by PCR methods as follows:
a. A first primer, neol3-529G, was prepared, that consists of the sequence (untranscribed strand) surrounding the unique Pstl site within the neo coding region and the 5' terminal sequence of the intron following the alternative H-ras exon IDX, Cohen et al., Cell 58:461 (1989), as it is found in the T24/EJ allele of the human H-ras gene, Cohen & Levinson, Nature 334:119 (1988). The complete nucleotide sequence of the neol3-529G primer is: The neo sequence is shown in upper case letters and the H-ras sequence is shown in lower case letters. The Pstl recognition sequence is underlined. Note that the last three nucleotides of the neo sequence are identical to the last three nucleotides of the alternative H-ras exon IDX. Cohen et al., Cell 58:461 (1989)
b. A second primer, neol3-567, was prepared, that consists of the sequence of the transcribed strand of the neo gene between the Ball cleavage site and the last nucleotide of the neo sequence in primer neol3-529G, followed by the sequence of the transcribed strand of the 3' terminus of H-ras intron D. Cohen et al., Cell 58:461 (1989). The complete nucleotide sequence of the neol3-567 primer is: The neo sequence is shown in upper case letters and the H-ras sequence is shown in lower case letters.
c. The primers neol3-529G and neol3-567 were used in the PCR method to amplify the intron located between IDX and exon 4, Cohen, et al., Cell 58:461 (1989), of the H-ras gene present in plasmid ilel 2N(G), Cohen & Levinson, Nature 334:119 (1988) (alternatively referred to as Ile12(G) in Cohen et al., Cell 58:461 (1989)). The resulting amplification product was subjected to partial digestion with PstI, and the fragment generated by cleavage at the Pstl recognition site within the neo coding sequence was isolated from a polyacrylamide gel following electrophoretic fractionation. The isolated fragment thus contains the entire intron D sequence that follows IDX in the H-ras gene of plasmid ile12N(G) inserted within the a Pstl-Ball fragment of the neo gene.

### 7. pNeol₃GΔS

This vector lacks the intron-internal Smal fragment of pNeol₃G (approximately 300 nts). It was constructed by digesting pNeol₃G with Smal and then self-ligating the larger Smal fragment.

### 8. pNeol₃G-65

This vector lacks the central portion of the H-ras intron present in vector pNeol3G, but retains the 5'-terminal 11 and 3'-terminal 54 nts of the intron. It was constructed by removal of the sequences between the 5'-proximal Smal site and the 3'-proximal EcoNI site of the intron through ligation of (i) the large Smal-Sstll fragment of vector pNeol₃G to (ii) the EcoNl-Sstll fragment of vector pNeol₃A (which vector is identical to pNeol₃G except for a G to A substitution at nucleotide position 4 of the intron) that contains the 3' portion of the H-ras intron, followed by neo and HBV sequences. The 5'-protruding end generated by EcoNI digestion was filled-in to allow for ligation of that end to the blunt-end generated by Smal cleavage.

### 9. pNeol₃G-101

This vector was constructed by adding a portion of the polylinker region of plasmid pML-UM20 to the 65 nt long intron of vector pNeol₃G-65, to produce a 101 nt long intron. The large Smal-Sstll fragment of vector pNeol₃G was ligated simultaneously to the 36 nt long Smal-Xhol fragment generated by digestion of pML-UM20 and to the pNeol₃A-derived EcoNl-Sstll fragment described above. The 5'-protruding end generated by Xhol digestion was filled-in to allow for blunt-end ligation. One of the resulting plasmids, pNeol₃G-101, has the filled-in Xhol site of the polylinker region fused to the Smal site of the pNeol₃G fragment (which regenerated the Xhol site), and the Smal-generated end of the polylinker region fused to the filled-in EcoNI end of the pNeol₃A fragment.

### 10. pNeol₃G-76

Vector pNeol₃G-76 contains a 76 nt long intron, and was derived from pNeol₃G-101 by deleting a portion of the pML-UM20-derived polylinker region within pNeol₃G-101. It was constructed by ligating the large Xhol-Sstll fragment of pNeol₃G-101 to the BamHl-Sstll fragment of pNeol₃G-101 that contains the 3' portion of the intron and the neo gene. The Xhol- and BamHI-generated 5' protruding ends were filled-in to allow for blunt-end ligation.

### 11. pNeol₃G-72*

Vector pNeol₃G-72* contains a 72 nt long intron, and was derived from pNeol₃G-101 by deleting a portion of the pML-UM20-derived polylinker region within pNeol₃G-101. The portion of the polylinker remaining in pNeol₃G-72* has the sequence 5'-GATCCCC-3'. This vector was constructed by ligating the large Smal-Sstll fragment of pNeol₃G to the BamHl-Sstll fragment of pNeol₃A-101 (which vector is identical to pNeol₃G-101 except for a G to A substitution at nucleotide position 4 of the intron) that contains the 3' portion of the intron and the neo gene. The 5' protruding end generated by BamHI cleavage was filled-in to allow for ligation to the Smal-generated blunt-end.

### 12. pNeo₃G-72

Vector pNeol₃G-72 also contains a 72 nt long intron, but differs from pNeol₃G-72* in that the polylinker-derived sequence of pNeol₃G-72* is replaced by the sequence 5'-TCGAGTC-3'. This vector was constructed as follows:
a. Intermediate vector 10.28.9 was generated through a four-part ligation. The four fragments were:
   i. The large EcoRl-Sstll fragment of pNeol₃A-101 that contains the HBV poly(A) region and pUC119 sequences.
   ii. The EcoRl-Xhol fragment of pNeol₃A-101 that contains the SVI6B-derived enhancer/promoter, the 5' portion of the neo gene, and the 5' terminal portion of the 101 nt long intron.
   iii. The EcoNl-Sstll fragment of pNeol₃A that contains the 3' portion of the intron and the neo gene.
   iv. A synthetic DNA fragment obtained by annealing two complementary oligonucleotides having the following sequences: These oligonucleotides were designed to leave a 5' overhang of sequence 5'-TCGA at one end and a 5' overhang of a single G nucleotide at the other end upon annealing. These overhangs are complementary to the 5' protruding ends generated by Xhol and EcoNI cleavage, respectively.
   Ligation of these four fragments generated vector 10.28.9 having an 85 nt intron and a G to A substitution at position 4 of the intron. This vector contains two closely spaced Xhol sites within the intron: one recreated through ligation of the Xhol-generated terminus of fragment 2 to the 5'-TCGA overhang of the synthetic fragment, and the other contained within the synthetic fragment (underlined).
b. pNeol₃G-72 was constructed by ligating the large Xhol-Sstll fragment of pNeol₃G-101 to the Xhol-Sstll fragment of 10.28.9 that contains the 3' portion of the intron and the neo gene. This construction removes the internal Xhol fragment from the intron, thus creating a 72 nt long intron, and the A to G substitution at nucleotide position 4 of the intron.

### 13. pNeol₃G-71 and pNeol₃G-70

These vectors were constructed through the same procedure as outlined above for pNeol₃G-72. In each case, however, the synthetic oligonucleotide sequences differed slightly from those used to construct pNeol₃G-72. To generate intermediate vector 10.28.5, the strand A oligonucleotide lacked the last nucleotide of the sequence shown above, while the strand B oligonucleotide lacked the first nucleotide of the strand B sequence shown. This resulted in an 84 nt long intron within this intermediate vector 10.28.5 and a 71 nt intron in the final pNeol₃G-71 vector. Similarly, intermediate vector 10.28.1 was created using a strand A oligonucleotide lacking the last two nucleotides of the strand A sequence shown above and a strand B oligonucleotide that lacked the first and third nucleotides of the strand B sequence shown above. This resulted in an 83 nt long intron in this intermediate vector 10.28.1 and a 70 nt long intron in the final vector pNeol₃G-70.

### 14. pNeol₃G-69

Vector pNeol₃G-69 contains a 69 nt long intron, and was derived from pNeol₃G-101 by deleting a portion of the pML-UM20-derived polylinker region within pNeol₃G-101. It was constructed by ligating the large Xhol-Sstll fragment of pNeol₃G-101 to the EcoNl-Sstll fragment of pNeol₃A that contains the 3' portion of the intron and the neo gene.

### EXAMPLE 2

### 1. Ras 2.2 Cell Line

Rat-1 cells, Seeburg, et al., Nature 312:71 (1984), were stably transfected by the calcium phosphate method, Graham & van der Eb, Virology 52:456 (1973), with plasmid pT24-10, Capon, et al. Nature 302:33 (1983), containing the transforming T24/EJ allele of the human H-ras gene. After two weeks of culturing in non-selective medium, foci of transformed cells appearing against a background of contact-inhibited cells were identified, further propagated, plated at low density in soft agar, from which individual colonies of transformed cells were isolated. One colony that showed a relatively high degree of transformation was designated Ras 2.2.

### 2. Transfection

Ras 2.2 cells were transfected in duplicate by the calcium phosphate method with the following DNAs, in the amounts indicated per 1.4 x 10⁶ cells:
1. 0.5 µg pRK-hyg, and
2. 10.0 µg pRSV-hGH, and
3. 2.0 µg of one of the following DNAs (neomycin-resistance vectors containing an intron-modified neomycin-resistance gene:
   a) pNeol₃G-71
   b) pNeol₃G-72
   c) pNeol₃G-72*
   d) pNeol₃G-76
   e) pNeol₃G-101
   f) pNeol₃G-ΔS

### 3. Selection

Cotransfected cells were transferred two days later to medium (Equal volumes of DMEM and F12 media, 5% fetal bovine serum, 2 mM glutamine) containing either 400 µg/ml of the neomycin analog G418 or 200 µg/ml hygromycin and thereafter fed every two days. G418-resistant colonies (G418^{r}) were counted after 20 days exposure to the drug and were pooled for further culturing. Hygromycin-resistant colonies (hyg^{r}) were counted after 10 days exposure to the selective drug and were pooled for further culturing. Cells in the hyg^{r} pool were passaged once and plated at variable densities into medium containing G418 at concentrations of either 350 µg/ml (hyg^{r} pools of cells transfected with pNeol₃G-71 or pNeol₃G-72) or 400 µg/ml (hyg^{r} pools of cells transfected with pNeol₃G-72*, pNeol₃G-76, pNeol₃G-101, or pNeol₃G-ΔS) and thereafter fed every two to four days. Resulting hygromycin/G418-resistant colonies (hyg^{r}/G418^{r}) were counted after two to four weeks exposure to G418 and were pooled for further culturing.

Figure 1 shows the number of drug resistant colonies obtained with the different neomycin-resistance vectors.

The data show that the optimal size intron is 71 or 72 nts, inasmuch as the pNeol₃G-71, pNeol₃G-72, and pNeol₃G-72* vectors containing introns of those lengths within the neomycin-resistance gene give rise to G418^{r} and hyg^{r}/G418^{r} colonies, but relatively fewer colonies than are obtained with the pNeol₃G-76 vector which contains a 76 nt long intron within the neomycin resistance gene.

### 4. Determining Levels of Human Growth Hormone (hGH)

### Expression in the Cotransfected Cell Cultures

Pools of G418, hyg^{r}, and hyg^{r}/G418 resistant cells at approximately equal cell densities were metabolically radiolabeled with ³⁵S-methionine and ³⁵S-cysteine and thereafter the levels of human growth hormone (hGH) present in the cell culture supernatants was determined by analyzing the supernatant proteins on sodium dodecyl sulfate (SDS) - polyacrylamide gels.

In addition, levels of hGH in the cell culture supernatants were determined by an ELISA radioimmunoassay using polyclonal anti-hGH antibody. Pools of drug-resistant cells were plated at a density of 1.5 x 10⁵ cells per well in a microtiter culture plate and allowed to grow to confluency. Cells were then harvested, counted, and the cell culture supernatants (conditioned medium) were assayed. Figure 2 shows for each of the different cotransfected cell types the calculated amount of hGH in the cell culture supernatant on a per cell basis (pg/cell). Those numbers were arrived at by dividing the amount of immunoreactive hGH in the cell culture supernatant from a given well of the microtiter plate (determined by ELISA) by the number of cells recovered from the well.

Consistent with the previous determination that the optimal size intron is 71 or 72 nts, the data in Figure 2 show that the highest levels of hGH are produced by cells cotransfected with the pNeol₃G-71, pNeol₃G-72, or pNeol₃G-72* vectors.

### 5. Determining Levels of Human Growth Hormone (hGH)

### Expression in Individual Cell Clones

Pools of G418 resistant cells transfected with either pNeol₃G-71 or pNeol₃G-ΔS were plated at very low density in 10 mm tissue culture dishes. Individual colonies (clones) were isolated and propagated for one passage, then the cells from each clone were harvested and plated at a density of 4 x 10⁸ cells per 60 mm tissue culture dish. After several hours, the cells were metabolically radiolabeled with ³⁵S-methionine and ³⁵S-cysteine and thereafter the levels of human growth hormone (hGH) present in the cell culture supernatants was determined by analyzing the supernatant proteins on sodium dodecyl sulfate (SDS) - polyacrylamide gels.

In addition, the levels of hGH produced by individual clones was determined by an ELISA radioimmunoassay using polyclonal anti-hGH antibody. The cells from each clone were plated at a density of 1.5 x 10⁵ cells per each well of a microtiter cell culture plate and allowed to grow to confluency. Cells were then harvested, counted, and the cell culture supernatants (conditioned medium) were assayed. Figure 3 shows for each of the different clones analyzed the number of cells, the amount of hGH in the cell culture supernatants (determined by ELISA), and the calculated amount of hGH produced per cell (pg/cell).

## Claims

1. A method for identifying or isolating a recombinant host cell that expresses high levels of a desired protein, which method comprises the steps of:
(1) transfecting a eukaryotic host cell with
(a) a selectable gene containing an intron that does not naturally occur within the selectable gene, wherein the intron is capable of being spliced in the host cell to provide messenger RNA encoding a selectable protein and wherein the presence of the intron in the selectable gene reduces the level of selectable protein produced from the selectable gene in the host cell; and
(b) a product gene encoding a desired protein; and
(2) identifying transfectants having the selectable phenotype conferred by the selectable protein.

2. The method of claim 1 wherein the selectable gene is from a prokaryotic source.

3. The method of claim 1 wherein the selectable gene is a hygromycin-resistance gene or a neomycin-resistance gene.

4. The method of claim 1 which further comprises transfecting the host cell with a second selectable gene.

5. The method of claim 4 wherein the second selectable gene is a hygromycin-resistance gene or a DHFR gene.

6. The method of claim 1 wherein the intron is from 55 to 85 nucleotides in length.

7. The method of claim 1 wherein the intron is from 65 to 75 nucleotides in length.

8. The method of claim 1 wherein the intron is present within the coding region of the selectable gene.

9. The method of claim 1 wherein the intron is present within the transcribed non-coding region of the selectable gene.

10. The method of claim 1 wherein the eukaryotic host cell is a mammalian cell.

11. The method of claim 10 wherein the mammalian cell is Chinese Hamster Ovary (CHO) cell, or a simian COS cell, or a human embryonic kidney 293 cell.

12. The method of claim 1 which further comprises recovering from the transfected host cell the protein encoded by the product gene.

13. A transfectant cell obtainable by the method of claim 1.

14. An isolated nucleic acid comprising a selectable gene and an intron within the transcribed region of the selectable gene, which intron does not naturally occur within the selectable gene, and which intron is of a length sufficient to reduce the level of selectable protein produced from the selectable gene in a host cell.

## Patentansprüche

1. Verfahren zur Identifizierung oder Isolierung einer rekombinanten Wirtszelle, die hohe Niveaus eines gewünschten Proteins exprimiert, welches Verfahren umfaßt die Schritte der:
(1) Transfektion einer eukaryotischen Wirtszelle mit
(a) einem selektierbaren Gen, das ein Intron enthält, welches nicht natürlicherweise innerhalb des selektierbaren Gens vorkommt, wobei das Intron imstande ist, in der Wirtszelle gespleißt zu werden, um mRNA bereitzustellen, die für ein selektierbares Protein kodiert, und wobei die Anwesenheit des Introns in dem selektierbaren Gen das Niveau an selektierbarem Protein, welches von dem selektierbaren Gen in der Wirtszelle produziert wird, verringert; und
(b) einem Produktgen, das für ein gewünschtes Protein kodiert; und
(2) Identifizierung von Transfektanten, die den selektierbaren Phänotyp aufweisen, welcher durch das selektierbare Protein verliehen wird.

2. Verfahren nach Anspruch 1, worin das selektierbare Gen aus einer prokaryotischen Quelle stammt.

3. Verfahren nach Anspruch 1, worin das selektierbare Gen ein Hygromycinresistenzgen oder ein Neomycinresistenzgen ist.

4. Verfahren nach Anspruch 1, welches ferner die Transfektion der Wirtszelle mit einem zweiten selektierbaren Gen umfaßt.

5. Verfahren nach Anspruch 4, worin das zweite selektierbare Gen ein Hygromycinresistenzgen oder eine DHFR-Gen ist.

6. Verfahren nach Anspruch 1, worin das Intron eine Länge von 55 bis 85 Nukleotiden aufweist.

7. Verfahren nach Anspruch 1, worin das Intron eine Länge von 65 bis 75 Nukleotiden aufweist.

8. Verfahren nach Anspruch 1, worin das Intron innerhalb des kodierenden Bereichs des selektierbaren Gens vorliegt.

9. Verfahren nach Anspruch 1, worin das Intron innerhalb des transkribierten nicht-kodierenden Bereichs des selektierbaren Gens vorliegt.

10. Verfahren nach Anspruch 1, worin die eukaryotische Wirtszelle eine Säugerzelle ist.

11. Verfahren nach Anspruch 10, worin die Säugerzelle eine Eierstockzelle des Chinesischen Hamsters (CHO) oder eine Affen-COS-Zelle oder eine Humanembryonieren-293-Zelle ist.

12. Verfahren nach Anspruch 1, welches ferner die Gewinnung des von dem Produktgen kodierten Proteins aus der transfizierten Wirtszelle umfaßt.

13. Transfektanten-Zelle, erhältlich nach dem Verfahren von Anspruch 1.

14. Isolierte Nukleinsäure, umfassend ein selektierbares Gen und ein Intron innerhalb des transkribierten Bereichs des selektierbaren Gens, welches Intron nicht natürlicherweise innerhalb des selektierbaren Gens vorkommt und welches Intron eine ausreichende Länge aufweist, um das Niveau an selektierbarem Protein, welches von dem selektierbaren Gen in einer Wirtszelle produziert wird, zu verringern.

## Revendications

1. Procédé permettant d'identifier ou d'isoler une cellule hôte de recombinaison qui produit en grande quantité une protéine voulue, lequel procédé comporte les étapes consistant :
1) à transfecter une cellule hôte eucaryote avec
a) un gène de sélection contenant un intron qui ne se trouve pas naturellement dans ce gène, lequel intron peut subir dans la cellule hôte l'épissage donnant un ARN messager codant une protéine de sélection, mais provoque, par sa présence dans le gène de sélection, une baisse de la quantité de protéine de sélection produite par le gène de sélection dans la cellule hôte, et
b) un gène de produit, codant une protéine voulue ; et
2) à identifier les cellules transfectées, présentant le phénotype de sélection qui leur est conféré par la protéine de sélection.

2. Procédé conforme à la revendication 1, dans lequel le gène de sélection est d'origine procaryote.

3. Procédé conforme à la revendication 1, dans lequel le gène de sélection est un gène de résistance à l'hygromycine ou un gène de résistance à la néomycine.

4. Procédé conforme à la revendication 1, qui comporte en outre le fait de transfecter la cellule hôte avec un second gène de sélection.

5. Procédé conforme à la revendication 4, dans lequel le second gène de sélection est un gène de résistance à l'hygromycine ou un gène de DHFR.

6. Procédé conforme à la revendication 1, dans lequel l'intron est long de 55 à 85 nucléotides.

7. Procédé conforme à la revendication 1, dans lequel l'intron est long de 65 à 75 nucléotides.

8. Procédé conforme à la revendication 1, dans lequel l'intron se trouve au sein de la région codante du gène de sélection.

9. Procédé conforme à la revendication 1, dans lequel l'intron se trouve au sein de la région transcrite non-codante du gène de sélection.

10. Procédé conforme à la revendication 1, dans lequel la cellule hôte eucaryote est une cellule de mammifère.

11. Procédé conforme à la revendication 10, dans lequel la cellule de mammifère est une cellule d'ovaire de hamster chinois (CHO), une cellule COS de singe ou une cellule 293 de rein d'embryon humain.

12. Procédé conforme à la revendication 1, qui comporte en outre le fait de récupérer, à partir de la cellule hôte transfectée, la protéine codée par le gène de produit.

13. Cellule transfectée que l'on peut obtenir par un procédé conforme à la revendication 1.

14. Acide nucléique isolé qui comporte un gène de sélection et un intron situé au sein de la région transcrite du gène de sélection, lequel intron ne se trouve pas naturellement dans ce gène et est assez long pour provoquer une baisse de la quantité de protéine de sélection produite par le gène de sélection dans une cellule hôte.


### SEQUENCE LISTING

### (1) GENERAL INFORMATION:

(i) APPLICANT: GENENTECH, INC.
(ii) TITLE OF INVENTION: METHODS FOR SELECTION OF RECOMBINANT HOST CELLS EXPRESSING HIGH LEVELS OF A DESIRED PROTEIN
(iii) NUMBER OF SEQUENCES: 5
(iv) CORRESPONDENCE ADDRESS:
   (A) ADDRESSEE: Genentech, Inc.
   (B) STREET: 460 Point San Bruno Blvd
   (C) CITY: South San Francisco
   (D) STATE: California
   (E) COUNTRY: USA
   (F) ZIP: 94080
(v) COMPUTER READABLE FORM:
   (A) MEDIUM TYPE: 5.25 inch, 360 Kb floppy disk
   (B) COMPUTER: IBM PC compatible
   (C) OPERATING SYSTEM: PC-DOS/MS-DOS
   (D) SOFTWARE: patin (Genentech)
(vi) CURRENT APPLICATION DATA:
   (A) APPLICATION NUMBER:
   (B) FILING DATE: 20 FEB 1992
   (C) CLASSIFICATION:
(vii) PRIOR APPLICATION DATA:
   (A) APPLICATION NUMBER: 07/677,045
   (B) FILING DATE: 29 MAR 1991
(viii) ATTORNEY/AGENT INFORMATION:
   (A) NAME: Hensley, Max D.
   (B) REGISTRATION NUMBER: 27,043
   (C) REFERENCE/DOCKET NUMBER: 693
(ix) TELECOMMUNICATION INFORMATION:
   (A) TELEPHONE: 415/266-1994
   (B) TELEFAX: 415/952-9881
   (C) TELEX: 910/371-7168

### (2) INFORMATION FOR SEQ ID NO:1:

(i) SEQUENCE CHARACTERISTICS:
   (A) LENGTH: 75 bases
   (B) TYPE: nucleic acid
   (C) STRANDEDNESS: single
   (D) TOPOLOGY: linear
(xi) SEQUENCE DESCRIPTION: SEQ ID NO:1:
   GAATTCGAGC TCGGTACCCG GGGATCCTCT AGAGTCGACC TGCAGGGGCC 50
   CTCGAGACGC GTGGCATGCA AGCTT 75

### (2) INFORMATION FOR SEQ ID NO:2:

(i) SEQUENCE CHARACTERISTICS:
   (A) LENGTH: 40 bases
   (B) TYPE: nucleic acid
   (C) STRANDEDNESS: single
   (D) TOPOLOGY: linear
(xi) SEQUENCE DESCRIPTION: SEQ ID NO:2:
   ATGAACTGCA GGACGAGGCA GCGCGGCTGT AGGTCTCCCG 40

### (2) INFORMATION FOR SEQ ID NO:3:

(i) SEQUENCE CHARACTERISTICS:
   (A) LENGTH: 33 bases
   (B) TYPE: nucleic acid
   (C) STRANDEDNESS: single
   (D) TOPOLOGY: linear
(xi) SEQUENCE DESCRIPTION: SEQ ID NO:3:
   CCAGCCACGA TCTGGGAAAG GAGGGATGGG ATC 33

### (2) INFORMATION FOR SEQ ID NO:4:

(i) SEQUENCE CHARACTERISTICS:
   (A) LENGTH: 20 bases
   (B) TYPE: nucleic acid
   (C) STRANDEDNESS: single
   (D) TOPOLOGY: linear
(xi) SEQUENCE DESCRIPTION: SEQ ID NO:4:
   TCGAGCCCTT TTCTCGAGTC 20

### (2) INFORMATION FOR SEQ ID NO:5:

(i) SEQUENCE CHARACTERISTICS:
   (A) LENGTH: 17 bases
   (B) TYPE: nucleic acid
   (C) STRANDEDNESS: single
   (D) TOPOLOGY: linear
(xi) SEQUENCE DESCRIPTION: SEQ ID NO:5:
   GGACTCGAGA AAAGGGC 17